# EUROPEAN PATENT SPECIFICATION

(11) **EP 2 853 211 B1**
(45) Date of publication and mention of the grant of the patent: **23.03.2022**
(21) Application number: 14182126.4
(22) Date of filing: 25.08.2014
(51) Int. Cl.: A61B 17/88

(54) **Bone treatment system**
Knochenbehandlungssystem
Système de traitement osseux

(30) Priority: 27.09.2013 JP 2013202748
(43) Date of publication of application: 01.04.2015
(73) Proprietor: Terumo Kabushiki Kaisha, Tokyo 151-0072 (JP)
(72) Inventor: HAYAKAWA, Koichi, Nakai-machi, Kanagawa 259-0151 (JP)
(74) Representative: Casalonga

(56) References cited:
- EP-A1- 0 284 672
- WO-A1-2008/114483
- WO-A1-2009/084109
- WO-A1-2013/074933
- US-A- 4 715 378

## Description

### Technical Field

The present invention relates to a bone treatment system for placing a bone filling material on a bone treatment site.

### Background Art

For example, if a person has osteoporosis due to rarefied bone density, in some cases, he or she may suffer a wrist fracture (distal radius fracture) by putting his or her hand on something when falling down. Currently, treatment for the distal radius fracture has adopted a plate fixing technique in which a plate (mainly a locking plate) is arranged so as to cross from a fractured main bone piece to a peripheral bone piece, and the plate and each bone are repositioned and fixed by a screw. However, since the plate is installed outside the bone, there is a possibility that the screw or an end portion of the plate damages surrounding tissues thereof (flexor tendon, extensor tendon, or the like) . In addition, there are some medical cases in which it is necessary to remove the screw from the plate in order to avoid complications such as tendon rupture and the like. In this case, it is necessary to carry out surgery by incising skin twice, when the plate is installed and when the screw is removed.

Therefore, in the treatment for the bone fracture, treatment which is less invasive and has fewer complications is required. For example, as disclosed in PTL 1, a method is proposed in which a bone filling material is placed inside the bones so as to promote osteosynthesis. A device disclosed in PTL 1 includes a balloon configured to have a bioabsorbable material in a distal portion of the device . During the treatment, the balloon filled with the bone filling material is placed inside the bone. If the balloon is absorbed into a living body after the placement, the bone filling material cured inside the balloon is exposed, and supports a bone fracture site from the inside of the bone instead of the plate or the screw in the previously described plate fixing technique. In this manner, the osteosynthesis is promoted. In WO2009084109 a unit for resetting bone fracture comprising an inlet tube (2) the front end of which is introduced into a space in the pyramid via a through hole formed in the pedicle of vertebral arch and which has an injection channel (8), a balloon (3) which is attached to the front end of the above-described inlet tube (2) and can be inserted in the deflated state and inflated in the pyramid, and a filling device (5) which is detachably attached to the base side of the inlet tube (2) and by which a filler (13) is injected into the balloon (3) via the injection channel (8) of the inlet tube (2), wherein the inlet tube (2) is provided with a discharge channel (10), in parallel to the injection channel (8) is disclosed.

### Citation List

### Patent Literature

PTL 1: JP-T-2006-505339 PTL2: WO2009084109 A1

### Summary of Invention

### Technical Problem

Incidentally, a device disclosed in PTL 1 adopts a configuration in which a bone filling material is caused to internally flow through the device when an inside of a balloon is filled with the bone filling material. In this case, the bone filling material is less likely to flow due to air present inside the device. In addition, since the air is mixed when the bone filling material is supplied, air bubbles or irregularities appear in the bone filling material left in a cured state. Consequently, there is a possibility that the bone filling material becomes brittle and cannot obtain sufficient strength.

The present invention is made in order to solve the above-described problem, and an object thereof is to provide a bone treatment system which can more favorably treat a bone by favorably discharging air internally present inside a device during filling of a bone filling material and by easily placing the bone filling material thereon.

### Solution to Problem

In order to achieve the above-described object, a bone treatment system according the present invention includes a shaft that has a lumen which enables a bone filling material to be placed on a bone treatment site to flow therethrough, a balloon that is filled with the bone filling material via the lumen disposed in a distal portion of the shaft, and a gas discharge portion that causes gas inside the lumen to flow in a direction toward a proximal end rather than the balloon, when the lumen is filled with the bone filling material.

According to the above-described configuration, the bone treatment system includes the gas discharge portion that causes the gas (air) inside the lumen to flow in the direction toward the proximal end rather than the balloon, when the lumen is filled with the bone filling material. In this manner, the air is removed from the lumen. Therefore, the bone filling material is likely to flow therein, and thus, smoothly fills the balloon. As a result, the bone filling material can be placed on a bone treatment site in a short time, thereby enabling a bone to be treated more favorably.

In this case, a configuration may be adopted in which the balloon is broken in a state of maintaining attachment to the shaft, thereby exposing the bone filling material.

The gas discharge portion prevents the bone filling material from being mixed with the air. Therefore, air bubbles or irregularities are less likely to appear in the bone filling material left in a cured state. Accordingly, it is possible to prevent the bone filling material from becoming brittle. When a deployment operation device breaks the balloon, the balloon can be easily collected.

In an example not claimed, the gas discharge portion may be configured to have a tube which can be arranged at a position where a distal portion inserted into the lumen moves close to the balloon, and the tube may have an opening portion formed in the distal portion, and a discharge path which is connected to the opening portion and can cause the gas to flow therethrough.

As described above, the gas discharge portion is configured to have the tube. In this manner, if the tube is inserted into the lumen, it is possible to extract the air from an opening portion on a distal side to a discharge path. Accordingly, it is possible to favorably discharge the gas from the distal side of the lumen.

Furthermore, the tube may be able to be detached from the lumen after the balloon is filled with the bone filling material.

In the bone treatment system, the tube is able to be detached therefrom. Therefore, when the deployment operation device is inserted after the filling of the bone fillingmaterial, the tube is no longer in the way, and thus, it is possible to easily insert the deployment operation device.

In addition, a configuration may be adopted in which the tube has rigidity which enables a hollow portion to be formed inside the bone filling material in response to curing of the bone filling material, and in which the bone treatment system further includes a needle member which is delivered via an inside of the hollow portion and breaks the balloon.

According to this configuration, it is possible to form the hollow portion by pulling out the tube from a cured bone filling material. Accordingly, it is possible to break the balloon by inserting the needle member via the hollow portion, and to place the bone filling material by exposing the bone filling material from the balloon.

In addition to the above-described configuration, the needle member may be formed to have an outer diameter which enables the needle member to be inserted into the discharge path of the tube.

Since the needle member is formed to have the outer diameter which enables the needle member to be inserted into the discharge path of the tube, the bone treatment system does not need to perform a pulling-out operation of the tube. Accordingly, it is possible to break the balloon by inserting the needle member.

Alternatively, it is also described and not claimed that the gas discharge portion be configured to have a discharge path which is formed inside a wall of the shaft configuring the lumen and which communicates with the lumen or the inside of the inflated balloon.

As described above, since the gas discharge portion is the discharge path which is formed inside the wall of the shaft, it is possible to easily discharge the air present in the lumen without using another member.

In this case, the discharge path may communicate with an opening portion formed on a distal surface of the shaft.

As described above, since the discharge path communicates with the opening portion formed on the distal surface of the shaft, the bone treatment system can discharge the air present in the lumen from the distal portion of the shaft. Accordingly, it is possible to minimize the remaining air on the distal side of the lumen. Therefore, it is possible to more favorably place the bone filling material.

In addition, the gas discharge portion may be configured to have a gas permeable member which blocks a liquid or a solid, while allowing the gas to be permeable therethrough by being disposed between the shaft and the balloon, in an attachment portion of the balloon in the shaft.

As described above, even when the gas discharge portion is configured to have the gas permeable member, it is possible to easily discharge the air present in the lumen via the gas permeable member.

In the present invention, the gas discharge portion may be configured to have a groove portion which is formed in the shaft, in an attachment portion of the balloon in the shaft.

As described above, even when the gas discharge portion is configured to have the groove portion formed in the shaft, it is possible to easily discharge the air present in the lumen via the groove portion.

Furthermore, the balloon is fixed to the shaft by being covered with a tubular fixing member, and the gas discharge portion may be configured to have a groove portion which is formed on an inner peripheral surface of the fixing member, and a fixing portion of the balloon which is formed in a shape enabling the fixing portion to come into contact with the groove portion along the groove portion.

As described above, even when the gas discharge portion is configured to have the groove portion formed in the fixing member, it is possible to easily discharge the air present in the lumen via the groove portion.

Furthermore, a configuration may be adopted in which the balloon is fixed to the shaft by a coil member being wound up, and the gas discharge portion may be configured to have a discharge path which is formed in such a manner that an inner surface of the balloon is separated from an outer surface of the shaft in a gap of a linear portion configuring the coil member.

As described above, the gas discharge portion is configured to have the discharge path which is formed in such a manner that the inner surface of the balloon is separated from the outer surface of the shaft in the gap of the linear portion forming the coil member. In this manner, it is possible to easily discharge the air present in the lumen via the discharge path.

### Advantageous Effects of Invention

According to the present invention, a bone treatment system can favorably discharge gas present in a device during filling of a bone filling material. Therefore, it is possible to easily and accurately place the bone filling material, thereby enabling a bone to be more favorably treated.

### Brief Description of Drawings

[Fig. 1] Fig. 1 is a side view illustrating an overall configuration of a bone treatment system according to an embodiment of the present invention.
[Fig. 2] Fig. 2A is a schematic view for illustrating a distal radius fracture to which the bone treatment system in Fig. 1 is applied, Fig. 2B is a first illustrating view for illustrating flow of treatment for the distal radius fracture in Fig. 2A, Fig. 2C is a second illustrating view for illustrating flow of the treatment subsequent to Fig. 2B, and Fig. 2D is a third illustrating view for illustrating flow of the treatment subsequent to Fig. 2C.
[Fig. 3] Fig. 3 is a side cross-sectional view of a placement device of the bone treatment system in Fig. 1.
[Fig. 4] Fig. 4A is a partial cross-sectional view illustrating an enlarged distal portion of the placement device in Fig. 3, and Fig. 4B is a partial cross-sectional view illustrating an inflated state of a filling balloon of the placement device in Fig. 3.
[Fig. 5] Fig. 5A is a first illustrating view for illustrating a placement measure of the bone filling material using the bone treatment system, and Fig. 5B is a second illustrating view for illustrating the placement measure of the bone filling material, subsequent to Fig. 5A.
[Fig. 6] Fig. 6A is a third illustrating view for illustrating the placement measure of the bone fillingmaterial, subsequent to Fig. 5B, and Fig. 6B is a fourth illustrating view for illustrating the placement measure of the bone filling material, subsequent to Fig. 6A.
[Fig. 7] Fig. 7A is a fifth illustrating view for illustrating the placement measure of the bone fillingmaterial, subsequent to Fig. 6B, and Fig. 7B is a sixth illustrating view for illustrating the placement measure of the bone filling material, subsequent to Fig. 7A.
[Fig. 8] Fig. 8A is a seventh illustrating view for illustrating the placement measure of the bone fillingmaterial, subsequent to Fig. 7B, and Fig. 8B is an eighth illustrating view for illustrating the placement measure of the bone filling material, subsequent to Fig. 8A.
[Fig. 9] Fig. 9 is a side cross-sectional view illustrating a placement device according to a first modification (not claimed) example.
[Fig. 10] Fig. 10A is a first side cross-sectional view illustrating a placement device according to a second (not claimed) modification example, Fig. 10B is a second side cross-sectional view illustrating the placement device according to the second modification example, and Fig. 10C is a third side cross-sectional view illustrating the placement device according to the second modification example.
[Fig. 11] Fig. 11A is a side cross-sectional view illustrating a placement device according to a third (not claimed) modification example, and Fig. 11B is a cross-sectional view taken along line XIB-XIB in Fig. 11A.
[Fig. 12] Fig. 12A is a side cross-sectional view illustrating a placement device according to a fourth (not claimed) modification example, and Fig. 12B is a side cross-sectional view illustrating a placement device according to a fifth (not claimed) modification example.
[Fig. 13] Fig. 13A is a side cross-sectional view illustrating a placement device according to a sixth modification example, and Fig. 13B is a side cross-sectional view illustrating a placement device according to a seventh modification example.
[Fig. 14] Fig. 14A is a side cross-sectional view illustrating a placement device according to an eighth modification example, Fig. 14B is a cross-sectional view taken along line XIVB-XIVB in Fig. 14A, and Fig. 14C is a front cross-sectional view illustrating a placement device according to a ninth modification example.

### Description of Embodiments

Hereinafter, a bone treatment system according to the present invention will be described in detail based on preferred embodiments and with reference to the accompanying drawings.

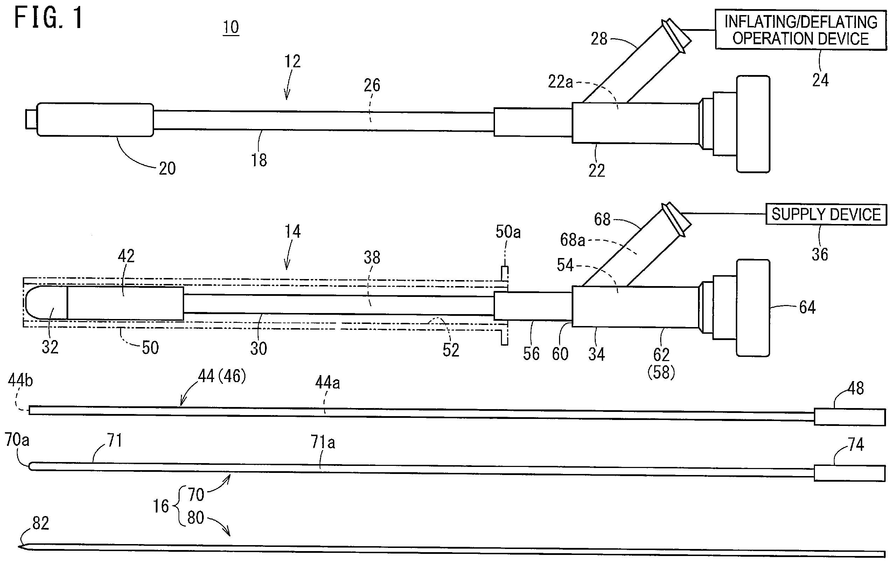

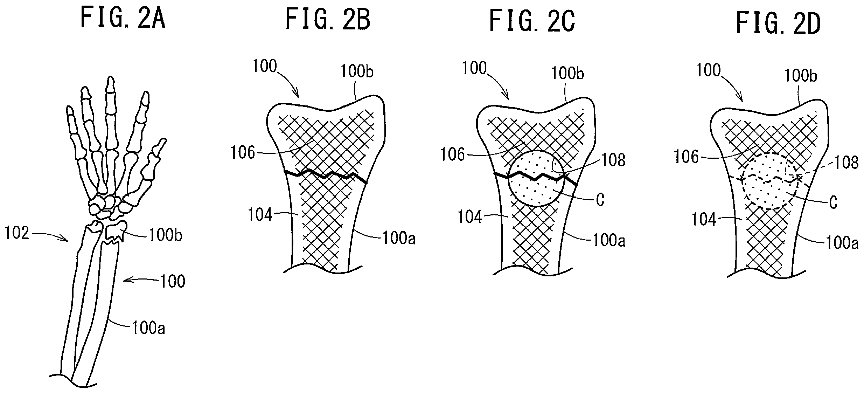

A bone treatment system 10 according to the present invention is a system in which multiple devices illustrated in Fig. 1 perform low invasive treatment on a patient's fracture site by using a bone filling material C (placement material: refer to Fig. 2C). That is, a surgeon shows a manual technique for placing the bone filling material C inside a bone by using the multiple devices of the bone treatment system 10 at a predetermined timing. The placed bone filling material C treats the bone with the lapse of time.

The "bone filling material" described herein represents those which can fill a bone treatment site in an initial stage (for example, liquid or paste) and are cured (for example, solidified or semi-solidified) with the lapse of time. Then, the "bone filling material" is a material which enables bone treatment (also including bone ameliorating such as osteosynthesis promotion and augmentation, in addition to osteosynthesis) to be performed on the material placed bone by means of fusion, absorption, substitution, organization, or the like.

A material for this bone filling material C is not particularly limited, but for example, it is possible to preferably use a polymethacrylate resin (PMMA) such as polymethyl methacrylate, α-type tricalcium phosphate (TCP), β-type TCP, and calcium phosphate cement (CPC) such as hydroxyapatite. In particular, the CPC is preferably used since the CPC enables the osteosynthesis by being solidified from paste within a relatively short time, being gradually joined to (absorbed in) the bone from a solidified state, and being eventually substituted with an autologous bone. Note that, a concept of the bone filling material C includes so-called "bone cement". The bone cement represents the above-described PMMA in a narrow sense, but includes the PMMA and the CPC in a broad sense.

For example, a treatment target using the bone treatment system 10 includes a wrist fracture (distal radius fracture) . As a matter of course, this bone treatment system 10 is not limited to the treatment for the distal radius fracture, and can be applied to treatment for other bone fracture sites or bone augmentation for osteoporosis. In addition, the treatment site is not limited to the inside of the bone, and the bone treatment system 10 can target various positions where the bone filling material C can be placed. Furthermore, the bone treatment system 10 can be applied to the bone treatment for animals in addition to humans, of course.

Note that, in the following description, when indicating directions of the respective devices of the bone treatment system 10, the left side in Fig. 1 is referred to as a "distal" side, and the right side in Fig. 1 is referred to as a "proximal" side.

Herein, in order to facilitate understanding of the bone treatment system 10 according to the present invention, an overview of the treatment for the distal radius fracture using the placement of the bone filling material C will be first described. As illustrated in Fig. 2A, a human body has a radius 100 located near a thumb and an ulna 102 located near a little finger, as bones of an upper limb on a further distal side than an elbow. The radius 100 includes a body portion 100a, a distal end portion 100b and a proximal end portion (not illustrated) which are located at both ends of the body portion 100a and are thicker than the body portion 100a. The osteoporosis of the radius 100 represents a state where bone tissues of a trabecular bone 106 on an inner side are lost as compared to a compact bone 104 on an outer peripheral surface side of the bone and thus bone density is rarefied.

The distal radius fracture occurs since the compact bone 104 in a connection portion between the body portion 100a and the distal end portion 100b receives a shock or the like and is destroyed (divided or cracked). A specific fracture state includes a closed fracture in which the divided distal end portion 100b is misaligned with the body portion 100a (also including Colles' fracture), an opened fracture, or an impression fracture in which the body portion 100a is forced into the distal end portion 100b.

As illustrated in Fig. 2B, in the treatment for the distal radius fracture, repositioning work is carried out in order to restore a position of the distal end portion 100b which is misaligned with the body portion 100a. This allows the body portion 100a and the distal end portion 100b to be mutually arranged at a normal position (hereinafter, referred to as a repositioned state).

In this repositioned state, positions of the body portion 100a and the distal end portion 100b are maintained, and as illustrated in Fig. 2C, a placement measure of the bone filling material C is executed using the bone treatment system 10. Although this placement measure will be described later, in a schematic configuration, the bone treatment system 10 forms a space 108 inside the repositioned radius 100, and carries out work for placing the bone filling material C inside the space 108. In this work, a filling balloon 32 (refer to Fig. 1) arranged in the space 108 is filled with the paste-like bone filling material C. Then, the filling material C is left in a cured state after a predetermined amount of time elapses, and is exposed from the filling balloon 32. Then, if the filling balloon 32 is collected from the space 108, only the cured bone filling material C is placed inside the space 108.

After the placement of the bone filling material C, a wrist is fixed by a cast or the like, and a fixing state thereof is continued for a predetermined time period. During this time period, the bone filling material C is absorbed by easily and reliably being in contact with the bone near the placement site inside the radius 100. The absorbed bone filling material C is gradually substituted with the autologous bone, and augments the inside of the radius 100, thereby joining the body portion 100a and the distal end portion 100b to each other. In the treatment for placing this bone filling material C, it is possible to minimize the influence on surrounding tissues of the fracture site. Therefore, as compared to a treatment method in the related art which fixes the fracture site using a metal plate or a screw, the treatment can be safely carried out.

Referring back to Fig. 1, next, a configuration of the bone treatment system 10 will be described in detail. The bone treatment system 10 is a therapeutic kit for implementing the above-described placement measure, that is, bone filling material placement therapy for curing the uncured bone filling material C and placing the cured bone filling material C inside the bone. This bone filling system 10 includes a space forming device 12, a placement device 14, and a deployment operation device 16.

Note that, the bone treatment system 10 can employ several devices (not illustrated) in addition to the above-described devices. For example, the bone treatment system 10 may include an opening / closing tool for incising or drilling into body tissues around a fracture site, a drill for drilling into the compact bone 104 of the radius 100, a cannula for building a guide path for the drill or the space forming device 12, and a fastener for maintaining a position of the repositioned bone. As these devices, known devices can be applied thereto, and thus, detailed description thereof will be omitted.

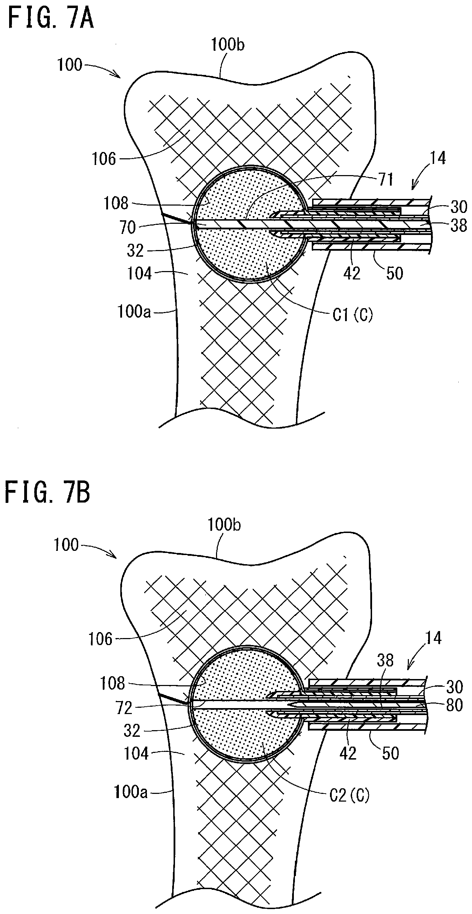

The space forming device 12 is a device for precedently forming the space 108 (refer to Fig. 7A) for placing the bone filling material C inside the repositioned radius 100. This space forming device 12 has a shaft 18, a space forming balloon 20 disposed on a distal side of the shaft 18, a hub 22 (grip portion) disposed on a proximal side of the shaft 18, and an inflating / deflating operation device 24 connected to the hub 22.

The shaft 18 is an elongated tubular member having a length by which the shaft 18 can reach the inside of the radius 100 from outside of the body.
In order for the shaft 18 to easily approach the inside of the radius 100 from outside of the body, it is preferable that the shaft 18 be configured to have a rigid material (for example, hard plastic or a metal material) . A lumen 26 for causing an inflating fluid to flow therethrough is formed inside the shaft 18 by penetrating the shaft 18 along an axial direction.

The space forming balloon 20 is a film member attached to a side surface of a distal portion of the shaft 18. The lumen 26 communicates with an inner side portion of the space forming balloon 20, and the inflating fluid can be introduced and discharged via this lumen 26. In this manner, the space forming balloon 20 is inflated radially outward on the side surface of the distal portion of the shaft 18, and shows a spherical shape in an inflated state. Note that, a shape of the inflated space forming balloon 20 is not limited to the spherical shape, and may be an elliptical shape in a side view, or may be a rounded shape as a whole. In addition, a horizontal cross-sectional shape of the inflated space forming balloon 20 is not limited to a circular shape (including a substantially circular shape), and may be the elliptical shape.

For example, the space forming balloon 20 is configured to have a non-elastic material. The non-elastic material includes fibrous porous film such as textile, knitted fabric, non-woven fabric, and a paper material, and additionally dense film such as non-fibrous porous film and a polymer sheet. Note that, the space forming balloon 20 may be configured to have an elastic material. For example, the elastic material includes various rubber materials such as natural rubber, butyl rubber, isoprene rubber, butadiene rubber, styrene-butadiene rubber, silicone rubber, various thermoplastic elastomers such as a polyurethane system, a polyester system, a polyamide system, an olefin system, and a styrene system, or mixtures thereof.

In addition, the inflating fluid supplied to the space forming balloon 20 is also not particularly limited. For example, a radiation ray (X-ray) contrast agent or physiological saline may be included. In particular, the X-ray contrast agent enables an operator to image an inflation degree of the space forming device 12 inside the radius 100 duringX-ray photography, and thus, is advantageously used from a viewpoint of setting a post-filling amount for the bone filling material C.

The hub 22 disposed on the proximal side of the shaft 18 is formed to have a diameter larger than that of the shaft 18 in order for a surgeon to easily operate the hub 22 when the surgeon shows his or her manual technique. In addition, the hub 22 is formed as a Y-type connector so as to connect the inflating / deflating operation device 24 thereto. An introduction space portion 22a which causes the inflating fluid supplied from the inflating / deflating operation device 24 to flow in the lumen 26 is disposed inside the hub 22.

For example, the inflating / deflating operation device 24 is configured to have a syringe and is connected to an introduction port 28 of the hub 22 (Y-type connector). The inflating / deflating operation device 24 has a function in which the inflating fluid is supplied to the shaft 18 side and further the supplied inflating fluid is suctioned by an operation of the surgeon. For example, if the inflating / deflating operation device 24 is the syringe, the surgeon performs a pushing operation of a plunger (not illustrated) so as to cause the inflating fluid to flow out from the syringe, and supplies the inflating fluid to the space forming balloon 20. In addition, after the inflating fluid is supplied, the surgeon takes off his or her hand from the plunger (or pulls out the plunger), thereby performing an operation for suctioning the inflating fluid.

Note that, the inflating / deflating operation device 24 may be configured so as to indicate a filling amount of the bone filling material C (alternatively, to indicate a supply amount itself of the inflating fluid), corresponding to an amount of the inflating fluid supplied to the space forming balloon 20. That is, a volume of the space 108 inside the radius 100 which is formed by the space forming device 12 is approximately equal to a volume of the supply amount of the inflating fluid. Therefore, the volume of the space 108 can be used as an index when filling the bone filling material C later. For example, the inflating / deflating operation device 24 may have a configuration in which the filling amount of the bone filling material C is jointly marked in a scale of the plunger of the syringe and an advanced position of the plunger is automatically marked when the inflating fluid is supplied to the maximum amount. In this manner, based on the indicated filling amount of the bone filling material C, it is possible to accurately fill the bone filling material C later.

The space forming balloon 20 of the space forming device 12 is left in a deflated state until the space forming balloon is inserted into the radius 100, and then, is inflated in response to the supply of the inflating fluid inside the radius 100. The space forming balloon 20, while being inflated, crushes bone tissues inside the radius 100. The trabecular bone 106 inside the radius 100 suffering from the osteoporosis is brittle, and thus, is relatively easily crushed by the inflated space forming balloon 20. In contrast, the space forming balloon 20 has sufficient strength. Therefore, there is no damage such as a rupture or a hole forming. In this manner, the space 108 is formed inside the radius 100 in response to the inflated space forming balloon 20.

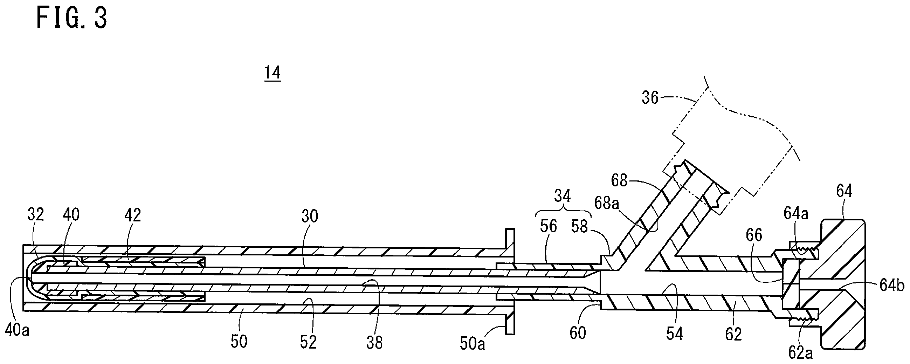

After the space forming device 12 is used, the placement device 14 of the bone treatment system 10 illustrated in Figs. 1 and 3 is used. The placement device 14 is a bone treatment device for placing the bone filling material C in the space 108 formed inside the radius 100. The placement device 14 includes the shaft 30, the filling balloon 32 disposed on the distal side of the shaft 30, a grip portion 34 disposed on the proximal side of the shaft 30, and a supply device 36 connected to the grip portion 34. In addition, the placement device 14 includes an air discharge tube 44 (gas discharge portion 46) which is inserted into the placement device 14 before the filling of the bone filling material C.

The shaft 30 is formed to have a length and a thickness which are substantially the same as those of the shaft 18 of the space forming device 12, and rigidity thereof is also set to be approximately the same. For example, a configuring material of the shafts 18 and 30 includes metal materials such as stainless steel, aluminum alloys, and copper-based alloys, and resin materials such as polyolefin, polyvinyl chloride, polyamide, polyamide elastomer, polyester, polyesterelastomer, polyurethane, polyurethane elastomer, polyimide, a fluorine resin, PEEK, polyethylene terephthalate, and the like.

A flow path 38 (lumen) for causing the paste-like bone filling material C to flow therethrough is formed inside the shaft 30 by penetrating the shaft 30 along the axial direction. This flow path 38 has an inner diameter sufficiently larger than that of the deployment operation device 16, and allows the deployment operation device 16 to pass therethrough even in a state where the deployment operation device 16 is fully filled with the bone filling material C. For example, the inner diameter of the flow path 38 is preferably set to 2 mm to 5 mm, and more preferably set to 2.5 mm to 4.5 mm.

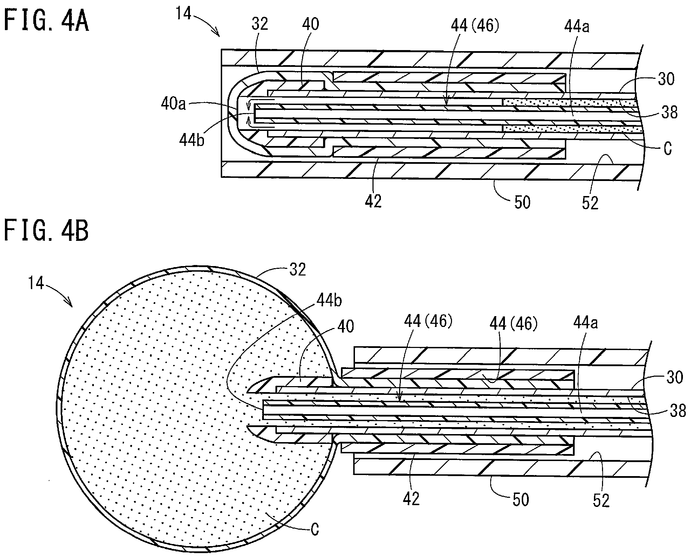

In addition, as illustrated in Figs. 3 and 4A, a tip 40 is attached to the distal portion of the shaft 30. The tip 40 is formed in a tapered shape in which a proximal portion side which is thicker than the outer diameter of the shaft 30 is fixed to an outer peripheral surface of the shaft 30 and is tapered toward the distal end from the proximal portion.

This tip 40 is configured to have a material which is more flexible than that of the shaft 30. A configuring material of the tip 40 is not particularly limited. However, for example, the tip 40 may be configured to have urethane resins or elastomeric materials such as polyurethane elastomer, polyester elastomer, and nylon elastomer. In addition, it is preferable that the tip 40 be configured to include a material to which an X-ray contrast-available imaging member can be attached, or an X-ray contrast-available material. This enables a surgeon to easily recognize a position of the distal portion of the placement device 14 when inserting the placement device 14 or when filling the bone filling material C.

An outer surface of the tip 40 is covered with the filling balloon 32 in a deflated state. The flexible tip 40 supports the filling balloon 32 from inside so as not to be damaged. In addition, the tip 40 supports the filling balloon 32 from inside in a state where the filling balloon 32 is inflated without any wrinkles. Therefore, the tip 40 can smoothly inflate the filling balloon 32. The lumen of the tip 40 having an opening portion 40a communicates with the flow path 38 of the shaft 30. The opening portion 40a ejects the bone filling material C flowed in the flow path 38, in a distal end direction. In thismanner, the opening portion 40a inflates the filling balloon 32 on the further distal side than the shaft 30.

The filling balloon 32 has elasticity, and is configured so that a deflated state of coming into contact with the outer surface of the tip 40 can be transferred to an inflated state of being separated from the tip 40 in response to the supplied bone filling material C. The proximal portion of the filling balloon 32 is fixed by a fixing tube 42. As illustrated in Fig. 4B, if the bone filling material C is supplied, the filling balloon 32 is inflated so as to show a spherical shape. In this inflated state, the distal end of the shaft 30 and the tip 40 are located on the proximal side inside the filling balloon 32.

The filling balloon 32 is configured to be more flexible than the space forming balloon 20, and becomes thin by being greatly stretched (elastically deformed) when inflated. Therefore, when inflated inside the radius 100, the filling balloon 32 has a three-dimensional shape which is appropriately deformed in response to the shape of the space 108. A configuring material of the filling balloon 32 is not particularly limited. However, it is preferable to form the filling balloon 32 by using a material having a certain degree of flexibility. For example, as this material, it is possible to use polyethylene, polypropylene, polybutene, ethylene-propylene copolymer, ethylene-vinyl acetate copolymer, ionomer, or mixtures of two or more type from among these such as polyolefin or soft polyvinyl chloride resins, thermoplastic resins such as polyamide, polyamide elastomer, polyester, polyester elastomer, polyurethane, and fluorine resins, silicone rubber, latex rubber, or the like.

The inflated filling balloon 32 is configured to be broken (ruptured) easily by the deployment operation device 16. The cured bone filling material C after filling is exposed in the space 108 of the radius 100 by breaking this filling balloon 32. In the filling balloon 32, the proximal portion is firmly fixed to the fixing tube 42. Accordingly, even after being broken, a state of being attached to the placement device 14 is maintained. As the placement device 14 is pulled out, the filling balloon 32 is collected from the inside of the radius 100.

The fixing tube 42 is attached so as to surround the proximal portion of the filling balloon 32, and fixes the filling balloon 32 by interposing the filling balloon 32 between the shaft 30 and the fixing tube 42. This fixing tube 42 has a predetermined length along the axial direction of the shaft 30 so as to firmly hold the proximal portion of the filling balloon 32.

Referring back to Fig. 3, when inserted into the radius 100, the placement device 14 includes the shaft 30, the filling balloon 32, and a tubular sheath 50 which protects the tip 40. The sheath 50 has an accommodation lumen 52 (accommodation portion) whose inner diameter is larger than the outer diameter of the fixing tube 42, and accommodates the shaft 30 or the filling balloon 32 so as to be slidable.

The sheath 50 is formed to have a length which is slightly longer than that of the shaft 30 (length from the tip 40 through the vicinity of the distal end of the grip portion 34). An operation plate 50a (sheath operation portion) protruding radially outward is disposed in the proximal portion of the sheath 50. The sheath 50 is moved forward and rearward relatively with the shaft 30 by a surgeon operating the operation plate 50a in the proximal end direction or in the distal end direction. The filling balloon 32 is exposed from the distal end of the sheath 50 by the rearward movement of the sheath 50.

The grip portion 34 of the placement device 14 is configured to have a Y-type connector, similar to the space forming device 12. An introduction space portion 54 communicating with the flow path 38 of the shaft 30 is formed inside the grip portion 34 by penetrating the grip portion 34. The grip portion 34 has a tubular distal end portion 56 formed to have the outer diameter which is smaller than the inner diameter of the accommodation lumen 52, and a tubular body portion 58 which is connected to the proximal end of the tubular distal end portion 56 and has a diameter larger than that of the tubular distal end portion 56. Therefore, a step 60 which opposes the proximal portion of the sheath 50 and regulates a rearward movement limit of the sheath 50 is formed between the tubular distal end portion 56 and the tubular body portion 58.

The tubular body portion 58 is a portion gripped by a surgeon when the placement device 14 is used, and is formed so as to coincide with an axial center of the shaft 30. The proximal side of this tubular body portion 58 is configured to serve as an insertion port 62 which enables the deployment operation device 16 or the air discharge tube 44 to be inserted into the flow path 38.

A male screw portion 62a is formed on the outer peripheral surface of the proximal end of the insertion port 62, and a guide member 64 having a female screw portion 64a is attached to this male screw portion 62a. The guide member 64 includes an insertion hole 64b which coincides with the axial center of the introduction space portion 54 in an attachment state thereof. This insertion hole 64b is formed to have the inner diameter which is slightly larger than the outer diameter of the deployment operation device 16 (mandrel 70). A valve body 66 is disposed between the guide member 64 and the grip portion 34. Whereas the valve body 66 allows and guides the deployment operation device 16 to be inserted, the valve body 66 has a function for preventing leakage of the bone filling material C.

In addition, an injection port 68 obliquely protruding in the proximal end direction is disposed on a side portion of the tubular body portion 58. The injection port 68 internally has an introduction path 68a for introducing the bone filling material C to the introduction space portion 54. An attachment portion (Luer-lock mechanism) to which the supply device 36 is detachably attached is disposed in the proximal portion thereof.

The supply device 36 is a device for filling of the bone filling material C, and for example, is configured to have a syringe, similar to the inflating / deflating operation device 24. A surgeon performs a pushing operation of a plunger, and accordingly the supply device 36 discharges the bone filling material C from the distal portion attached to the attachment portion of the injection port 68. In this manner, the bone filling material C is supplied to the flow path 38 of the shaft 30 via the introduction path 68a and the introduction space portion 54.

In an example not claimed, the air discharge tube 44 (gas discharge portion 46) is arranged inside the flow path 38 of the shaft 30 before the bone filling material C is supplied. As illustrated in Fig. 1, the air discharge tube 44 is an elongated tubular member which is linearly formed, and has a function for discharging the air (gas) of the flow path 38 to the proximal side rather than the filling balloon 32 when the bone filling material C is supplied.

The air discharge tube 44 is inserted into the insertion port 62 from the insertion hole 64b of the guide member 64 via the valve body 66, and is inserted to the flow path 38 through the introduction space portion 54. Note that, the placement device 14 may be left in a state where the air discharge tube 44 is inserted into the flow path 38 in advance when the bone treatment system 10 is delivered as a product.

An air discharge path 44a for the air is formed to penetrate along the axial direction inside the air discharge tube 44. A distal opening portion 44b communicating with the air discharge path 44a is disposed on the distal surface of the air discharge tube 44, and the air discharge tube 44 can cause the air to flow from the distal opening portion 44b to the air discharge path 44a. Note that, a forming location for an opening portion communicating with the air discharge path 44a is not particularly limited. The opening portion may be formed on a distal side lateral surface of the air discharge tube 44, or may be disposed at multiple locations along the axial direction of the air discharge tube 44.

The air discharge tube 44 has a predetermined axial length so as to be inserted into a position where the air discharge tube 44 moves close to a distal end inner surface of the deflated filling balloon 32. In addition, the air discharge tube 44 may include the air discharge path 44a which can discharge the air, and may be formed so that the outer diameter thereof is considerably small (thin).

It is preferable that an operation portion 48 whose diameter is larger than that of the air discharge tube 44 be attached to the proximal portion of the air discharge tube 44. The operation portion 48 facilitates a forward movement operation of the air discharge tube 44, and can regulate an insertion amount of the air discharge tube 44. For example, the operation portion 48 can be configured so that the operation portion 48 is formed to have the outer diameter which matches the inner diameter of the insertion hole 64b of the guide member 64, and that the distal opening portion 44b of the air discharge tube 44 is arranged close to the filling balloon 32 by fitting the operation portion 48 into the insertion hole 64b. This can prevent the air discharge tube 44 formed to be thin from coming into contact with the filling balloon 32 and causing damage to the filling balloon 32 or the air discharge tube 44.

It is preferable that a material configuring the air discharge tube 44 have a degree of rigidity in which the air discharge path 44a is not crushed due to supply pressure of the bone filling material C. In this case, for example, it is possible to preferably apply those which are included in the configuring material of the shafts 18 and 30. However, it is preferable to confirm a position of the distal portion of the air discharge tube 44 by X-ray photography. Accordingly, if the X-ray contrast is not available at all when the distal portion is used alone, it is preferable to dispose a marker made of gold or platinum in the distal portion. In addition, it is preferable that a surface of the air discharge tube 44 have a lubricating performance in order to facilitate a relative movement with respect to the cured bone filling material C. Alternatively, if necessary, lubricant (lubricant for an insertion member) may be coated thereon. For example, the lubricant includes silicone oil or the like.

In a state where the air discharge tube 44 is arranged in the flow path 38, the bone filling material C is supplied from the supply device 36 and flows in a distal direction of the shaft 30. At this time, the air inside the shaft 30 is oriented in the distal direction by the supply pressure from the bone filling material C, is caused to flow from the distal opening portion 44b of the air discharge tube 44 to the air discharge path 44a, and is discharged to the proximal portion of the shaft 30 (proximal side rather than the filling balloon 32). Accordingly, the bone filling material C is smoothly guided in the distal direction, and fills the inside of the filling balloon 32. The filling balloon 32 is in an inflated state as illustrated in Fig. 4B, in the distal portion of the shaft 30, in response to the filling of the bone filling material C. Then, the bone filling material C after the filling is left in a state of being cured to a placement-available degree inside the radius 100, if a predetermined time period (for example, approximately 5 minutes to 15 minutes) elapses.

Referring back to Fig. 1, the deployment operation device 16 is a device for exposing the above-described cured bone filling material C in the space 108 of the radius 100. This deployment operation device 16 is configured to have the mandrel 70 (metal core) which is an insertion member to be inserted before the bone filling material C is cured, and a needle member 80 to be inserted after the bone filling material C is cured and the mandrel 70 is pulled out.

The mandrel 70 is an elongated rod member which is linearly formed. After the filling of the bone filling material C (after the filling balloon 32 is inflated), the air discharge tube 44 is pulled out from the flow path 38 by a surgeon, and the mandrel 70 is inserted into the placement device 14 instead of the air discharge tube 44. In this manner, the mandrel 70 forms a passage 72 inside the cured bone filling material C.

The mandrel 70 is inserted until the mandrel 70 comes into contact with the inner surface of the inflated filling balloon 32. As a result, the passage 72 is formed in the hollow portion having a linear shape extending from the proximal portion of the grip portion 34 (inserting port 62) to the inflated filling balloon 32. Therefore, the mandrel 70 is set so that the longitudinal length is longer than that of the placement device 14 and the outer diameter is smaller than the inner diameter of the flow path 38 of the placement device 14.

It is preferable that the elongated portion 71 of the mandrel 70 be configured to have an X-ray contrast-available material so as to easily recognize a relative position of the mandrel 70 with respect to the filling balloon 32. In addition, it is preferable that a distal portion 70a of the mandrel 70 be formed in a round angle so as not to be broken even when coming into contact with the inflated filling balloon 32.

When inserted, an orientation of the mandrel 70 is guided by the guide member 64 attached to the grip portion 34, and the valve body 66. In addition, in a state where the distal portion 70a reaches the filling balloon 32, the proximal portion is held by the valve body 66. In this manner, the orientation is excellently maintained until the bone filling material C is cured. In order for the mandrel 70 to facilitate a forward movement operation, it is preferable that an operation portion 74 whose diameter is larger than that of the mandrel 70 be attached to the proximal portion of the elongated portion 71. The operation portion 74 maybe formed so as to be held by the insertion hole 64b in an insertion state of the mandrel 70 by forming the operation portion 74 to have the outer diameter which coincides with the inner diameter of the insertion hole 64b of the guide member 64.

If the predetermined amount of time elapses after the mandrel 70 is inserted into the placement device 14 and the passage 72 is formed in the cured bone filling material C, the mandrel 70 is pulled out from the placement device 14 by a surgeon. The outer peripheral surface of the elongated portion 71 is smoothly formed. The mandrel 70 can be relatively easily pulled out by being slightly vibrated or rotated. Note that, the elongated portion 71 may be coated with a lubricant 71a (lubricant for the insertion member) which facilitates a relative movement for the cured bone filling material C. For example, the lubricant 71a includes silicone oil or the like. After the mandrel 70 is pulled out, the needle member 80 is inserted into the passage 72.

Similar to the mandrel 70, the needle member 80 is formed in an elongated linear shape, and has a sharp needle tip 82 in the distal portion. The needle member 80 is formed to have the outer diameter which is slightly smaller than the outer diameter of the mandrel 70, and can smoothly pass through the passage 72 of the bone filling material C which is formed by the mandrel 70. The needle tip 82 of the needle member 80 moves the placement device 14 in the distal end direction and comes into contact with the inflated filling balloon 32. In this manner, the needle tip 82 can easily break the filling balloon 32. Note that, in order to increase sliding performance of the needle member 80, similar to the mandrel 70, the outer peripheral surface of the needle member 80 may be coated with the lubricant 71a.

A configuring material of the mandrel 70 or the needle member 80 is not particularly limited. For example, themandrel 70 or the needle member 80 may be configured to have a metal material such as stainless steel. In addition, it is preferable that the needle member 80 also be X-ray contrast-available. In this manner, when the forward movement operation of the needle member 80 is performed, it is possible to recognize that the needle tip 82 reaches the filling balloon 32 and breaks the filling balloon 32.

The bone treatment system 10 according to the present embodiment is basically configured as described above. Next, an operation effect of the bone treatment system 10 will be described.

Hereinafter, as an example of bone treatment using the bone treatment system 10, treatment for a distal radius fracture which is performed by placing the bone filling material C will be described. As illustrated in Fig. 2B, a surgeon repositions a fracture site of the radius 100 before implementing a placement measure, and arranges a normal positional relationship between the tubular body portion 100a and the distal end portion 100b. Then, the placement measure is implemented for the repositioned radius 100. Note that, in the subsequent placement measure, X-ray photography is carried out, and a state of the fracture site of the radius 100 is confirmed on a real-time basis by using an X-ray photographed image.

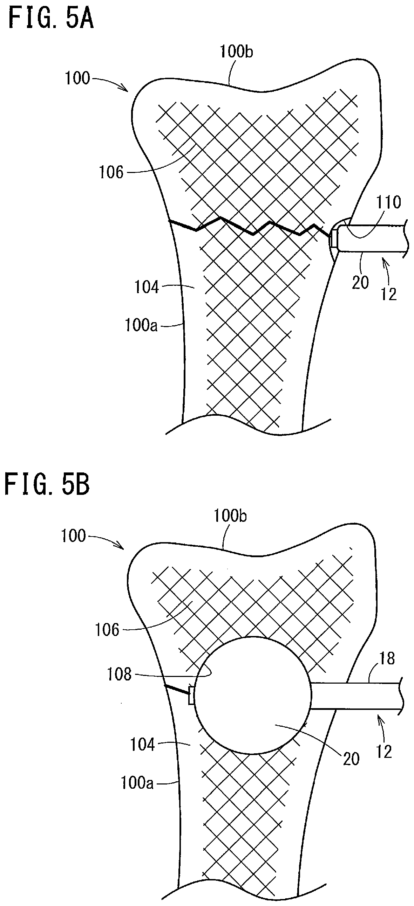

As illustrated in Fig. 5A, in the placement measure, the space forming device 12 is first used. The surgeon holds a repositioned state and incises the skin. Then, the surgeon forms a hole 110 for inserting the space forming device 12 and the placement device 14 with respect to the compact bone 104 of the radius 100 by using a drill or the like. It is preferable that the hole 110 be drilled along a site cracked due to the fracture. In this manner, it is possible to easily insert the space forming device 12 or the placement device 14 into the hole 110 later. Note that, a drilling direction of the hole 110 with respect to the radius 100, in other words, an inserting direction of the space forming device 12 or the placement device 14 is not particularly limited. The direction may be appropriately set depending on fracture conditions.

After the hole 110 is formed, the space forming device 12 is inserted into the hole 110. At this time, the space forming balloon 20 is left in a deflated state, and thus, the distal portion of the space forming device 12 is easily inserted into the radius 100. In addition, the trabecular bone 106 inside the radius 100 is brittle due to the osteoporosis . Accordingly, the space forming device 12 is allowed to enter the inside of the radius 100.

As illustrated in Fig. 5B, if the surgeon inserts the distal end portion of the space forming device 12 into a rear portion (compact bone 104 on a side opposite to the hole 110) of the radius 100, the surgeon inflates the space forming balloon 20 by supplying the inflating fluid. The space forming balloon 20 has sufficient strength, and thus, crushes the trabecular bone 106 which becomes brittle due to supplied pressure of the inflating fluid when the space forming balloon 20 is inflated. In this manner, the space 108 is formed inside the radius 100 in response to an inflated amount (volume) of the space forming balloon 20. After the space 108 is formed, the space forming balloon 20 is deflated and the space forming device 12 is pulled out from the inside of the radius 100.

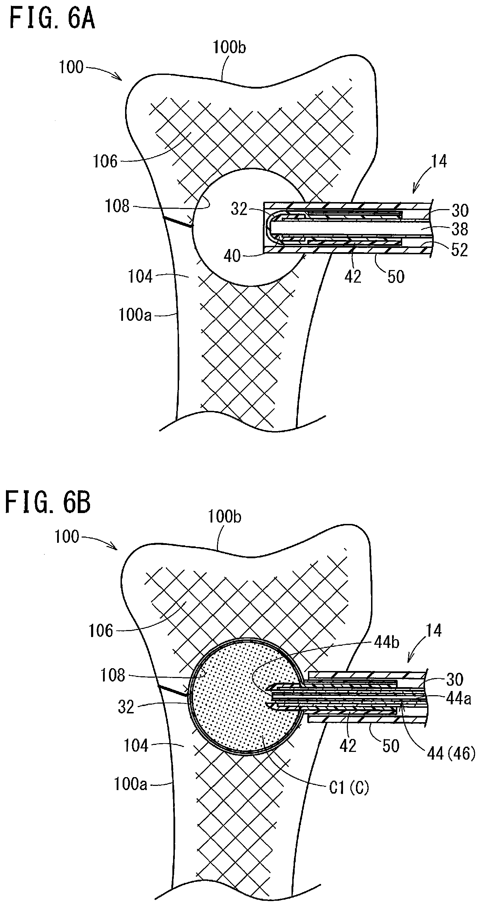

Next, as illustrated in Fig. 6A, the placement device 14 is used instead of the space forming device 12. In a state where the shaft 30 and the filling balloon 32 are accommodated in the accommodation lumen 52 of the sheath 50, the surgeon causes the placement device 14 to move forward to the radius 100 and inserts the placement device 14 into the hole 110 (refer to Fig. 5A). At this time, the sheath 50 is also inserted into the radius 100. In this manner, it is possible to avoid damage to the filling balloon 32. In addition, it is possible to recognize an insertion amount of the device with respect to the radius 100 from outside of the body. Note that, when the placement device 14 is inserted, the sheath 50 may be used for alignment of the hole 110, outside the radius 100. The shaft 30 or the filling balloon 32 may be inserted into the radius 100 by being caused to move forward with respect to the sheath 50.

If the distal end portion of the placement device 14 is inserted into the space 108, the operation plate 50a of the sheath 50 is caused to move rearward in the proximal end direction. Then, the distal portion of the shaft 30 (deflated filling balloon 32) is exposed inside the space 108. The sheath 50 is caused to perform a rearward movement operation until the proximal portion comes into contact with the step 60 of the grip portion 34. In this manner, the filling balloon 32 can be reliably inflated.

After the sheath 50 is moved rearward, the surgeon inserts the air discharge tube 44 through the insertion port 62 of the placement device 14, and delivers the distal portion to a portion close to the filling balloon 32. In this manner, it is possible to extract the air present near the distal end of the shaft 30 from the distal opening portion 44b of the air discharge tube 44. After the air discharge tube 44 is arranged, the surgeon operates the supply device 36. As illustrated in Fig. 6B, the bone filling material C is supplied to the filling balloon 32 through the flow path 38 of the shaft 30. Note that, in the following description, the reference number C1 will be given to a paste-like bone filling material, and the reference number C2 will be given to a bone filling material left in a cured state (state of being cured to such an extent so as not to be disintegrated by at least body fluid, or a more cured state) . When the bone filling material C1 is supplied, the air inside the flow path 38 is discharged to the outside of the body via the air discharge path 44a of the air discharge tube 44.

The filling balloon 32 is inflated at the distal end of the shaft 30 corresponding to a shape of the space 108 by the bone filling material C1 filling the inside of the filling balloon 32. It is preferable to set an inflated amount of the filling balloon 32 to such an extent so as to substantially coincide with or be slightly smaller than the volume of the space 108. This prevents the filling balloon 32 from being strongly pressed between a filling portion of the bone filling material C2 and the inner surface of the radius 100 due to the excessive filling of the bone filling material C1. Therefore, it is possible to smoothly collect the filling balloon 32 later.

After the filling balloon 32 is inflated, the air discharge tube 44 is pulled out, and the mandrel 70 (deployment operation device 16) is inserted through the insertion port 62 of the placement device 14, instead of the air discharge tube 44. The surgeon causes the mandrel 70 to perform the forward movement operation in the distal end direction via the flow path 38 of the placement device 14. As illustrated in Fig. 7A, the distal portion 70a comes into contact with the inner surface of the filling balloon 32, thereby stopping the forward movement. In the mandrel 70, a position of the distal portion 70a is confirmed during X-ray photography. Accordingly, it is possible to prevent the mandrel 70 from being unnecessarily pushed out to the filling balloon 32 and breaking the filling balloon 32 before the bone filling material C1 is cured.

After the mandrel 70 is inserted, the surgeon causes the bone filling material C1 to be cured for a predetermined waiting time. For example, if the bone filling material C is a curable CPC, the bone filling material C is cured within approximately 10 minutes to such an extent so as not to be easily disintegrated due to the body fluid. Therefore, a patient does not feel a heavy burden.

After the predetermined amount of time elapses, the surgeon performs an operation for pulling out the mandrel 70 from the cured bone filling material C2. The linearly formed mandrel 70 can be smoothly pulled out along the axial direction of the placement device 14 without being disadvantageously caught on the bone filling material C2.

If the mandrel 70 is pulled out, as illustrated in Fig. 7B, the passage 72 is formed inside the cured bone filling material C2. The passage 72 leads to the insertion port 62 of the placement device 14 into which the mandrel 70 is inserted. The surgeon inserts the needle member 80 through the insertion port 62, thereby enabling the needle member 80 to move forward along the passage 72.

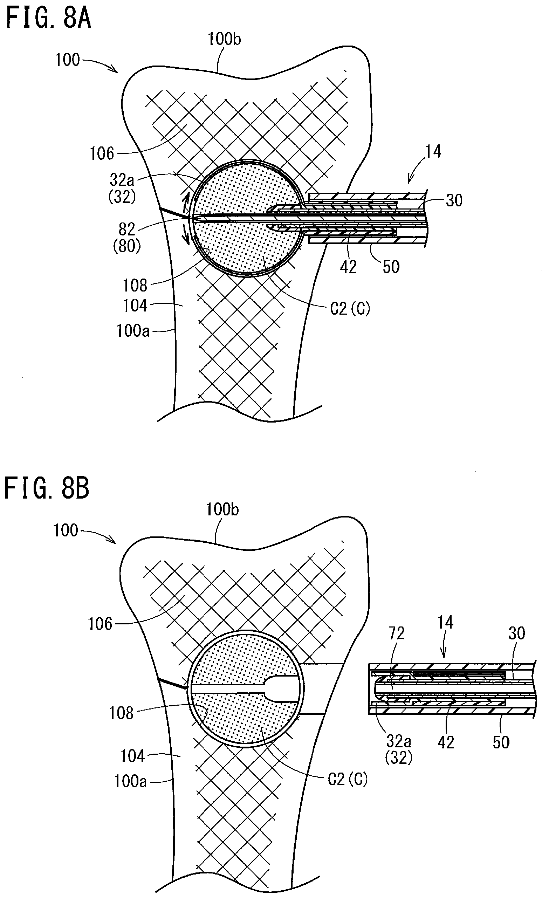

The passage 72 is formed in a linear shape extending to the inner surface of the filling balloon 32. Accordingly, the surgeon can smoothly perform the forward movement operation of the needle member 80 along the passage 72, and can easily cause the needle tip 82 to come into contact with a substantially center position of the filling balloon 32 opposing the passage 72. This contact of the needle tip 82 causes the filling balloon 32 to be broken from the rear side of the space 108 as illustrated in Fig. 8A. Note that, Fig. 8A illustrates a state immediately after the filling balloon 32 is ruptured. The needle tip 82 pierces the most stretched portion within the inflated filling balloon 32. Accordingly, this prompts the filling balloon 32 to be actively ruptured. In this manner, the cured bone filling material C2 is exposed inside the space 108.

The flexible filling balloon 32 is broken from the distal side (distal portion side) of the placement device 14. Accordingly, the filling balloon 32 is actuated so that a broken film portion (ruptured piece 32a) is moved to the proximal portion side which is fixed to the shaft 30. That is, the ruptured piece 32a is moved inside the bone filling material C2 and comes close to the shaft 30 side, thereby actively exposing the bone filling material C2. In addition, the placement device 14 can easily collect the filling balloon 32 (ruptured piece 32a) which is moved to the proximal portion side.

After the bone filling material C2 is exposed, as illustrated in Fig. 8B, the needle member 80 is pulled out from the passage 72, and the placement device 14 is further pulled out from the inside of the radius 100. In this manner, the cured bone filling material C2 is placed in the space 108 of the radius 100.

The cured bone filling material C2 is absorbed in the bone near the inner periphery of the radius 100, and then is gradually substituted with the autologous bone. As a result, in the radius 100, the tubular body portion 100a and the distal end portion 100b are joined to each other.

As described above, the bone treatment system 10 according to the present embodiment includes the gas discharge portion 46 which causes the air inside the flow path 38 to flow in the direction toward the proximal end rather than the filling balloon 32. Therefore, since the air is extracted from the flow path 38, it is possible to smoothly fill the filling balloon 32 with the bone filling material C. In addition, it is possible to prevent the bone filling material C from being mixed with the air. Therefore, air bubbles or irregularities are less likely to appear in the cured bone filling material C. Accordingly, it is possible to prevent the bone filling material C from becoming brittle. The filling balloon 32 can be easily collected. As a result, it is possible to more favorably treat a fracture site by easily and accurately placing the cured bone filling material C2 with respect to the space 108 of the radius 100.

In this case, since the gas discharge portion 46 is configured to have the air discharge tube 44, it is possible to favorably discharge the air inside the flow path 38 only by arranging the air discharge tube 44 inside the flow path 38.

Note that, the bone treatment system 10 according to the present invention can adopt various modification examples and application examples without being limited to the above-described configurations. For example, a configuration for breaking a portion of the filling balloon 32 is not particularly limited, and can employ various configurations. For example, as an operation member used instead of the above-described needle member 80, a device including a heating portion in the distal portion may be inserted into the passage 72. In this manner, the heating portion may cut off the portion of the filling balloon 32 and may rupture the filling balloon 32. Alternatively, as another operation member, a device including a scissoring mechanism in the distal portion may be inserted into the passage 72. In this manner, the scissoring mechanism may cut off the portion of the filling balloon 32 and may rupture the filling balloon 32.

Hereinafter, some modification examples of the bone treatment system 10 will be described in detail. Note that, in the following description, the same reference numerals are given to configurations the same as those of the bone treatment system 10 according to the present embodiment or configurations having the same functions, and detailed description thereof will be omitted.

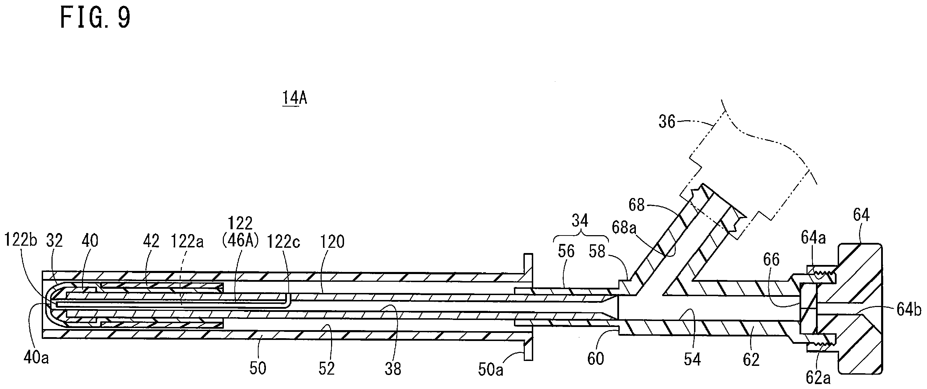

A gas discharge portion 46A of a placement device 14A according to a first modification example (not claimed) illustrated in Fig. 9 has a configuration in which an air discharge tube 122 is fixed to a lateral surface at an axially intermediate position of a shaft 120. The air discharge tube 122 has an air discharge path 122a, a distal opening portion 122b which communicates with the air discharge path 122a, and a proximal opening portion 122c. The proximal portion thereof is attached to a wall of the shaft 120. The proximal opening portion 122c is open outward from the shaft 120. Therefore, the air discharge tube 122 can extract the air from the distal opening portion 122b and can discharge the air to the proximal opening portion 122c, when the bone filling material C is supplied.

Note that, for example, if the air discharge tube 122 is configured to have a relatively flexible material and the air discharge tube 122 is arranged along a side (inner peripheral surface of the shaft 120) of the flow path 38, the mandrel 70 may be immediately inserted after the bone filling material C is supplied. Since the air discharge tube 122 is thin, the mandrel 70 is no longer in the way and performs a forward movement operation.

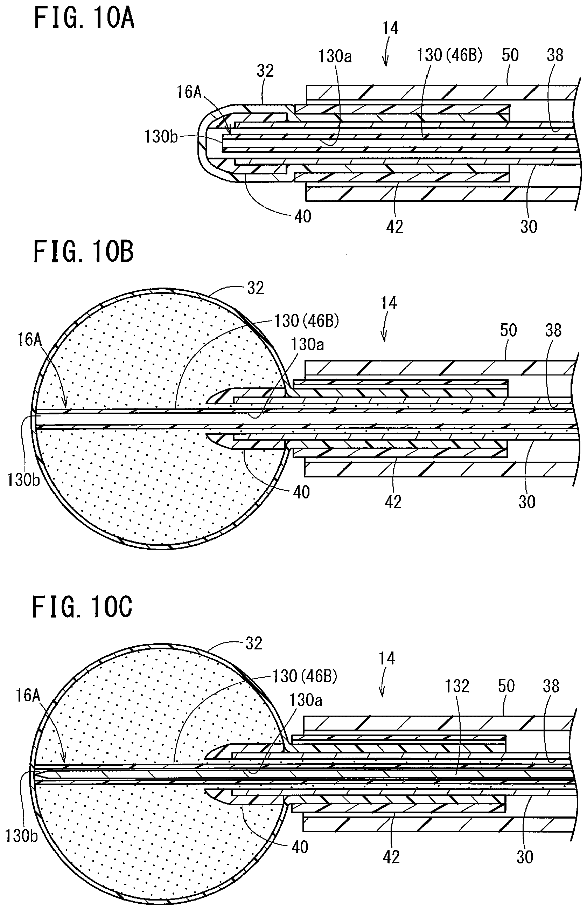

Amandrel 130 (deployment operation device 16A) according to a second modification example (not claimed) illustrated in Figs. 10A to 10C is formed in a tubular shape which has a penetrating path 130a (air discharge path) along the axial direction, and the mandrel 130 itself is configured as a gas discharge portion 46B. The penetrating path 130a communicates with a distal opening portion 130b formed on the distal surface of the mandrel 130. In addition, the deployment operation device 16A may include a needle member 132 having a size which enables the needle member 132 to move through the penetrating path 130a of the mandrel 130.

As illustrated in Fig. 10A, the mandrel 130 is inserted into the flow path 38 by a surgeon before the filling of the bone filling material C, and the distal portion thereof is arranged at a position close to the inner surface of the filling balloon 32. If the filling of the bone filling material C is performed in this state, it is possible to discharge the air of the flow path 38 via the penetrating path 130a. After the bone filling material C inflates the filling balloon 32, as illustrated in Fig. 10B, the mandrel 130 is moved forward and is brought into contact with the inner surface of the inflated filling balloon 32. Then, in the cured state of the bone filling material C, as illustrated in Fig. 10C, the needle member 132 is moved forward along the penetrating path 130a without pulling out the mandrel 130 therefrom. In this manner, it is possible to easily break the filling balloon 32.

Note that, even when the mandrel 130 according to the second modification example is used, as a matter of course, an operation for inserting the needle member 132 may be performed by pulling out the mandrel 130 therefrom after the bone filling material C is cured. In addition, even when a configuration is adopted in which the needle member 132 is formed of a hollow member having an air discharge path in the axial direction and is arranged in the penetrating path 130a of the mandrel 130 in advance, it is possible to favorably discharge the air. In this case, it is possible to quickly perform a rupturing operation on the filling balloon 32 by using the needle member 132.

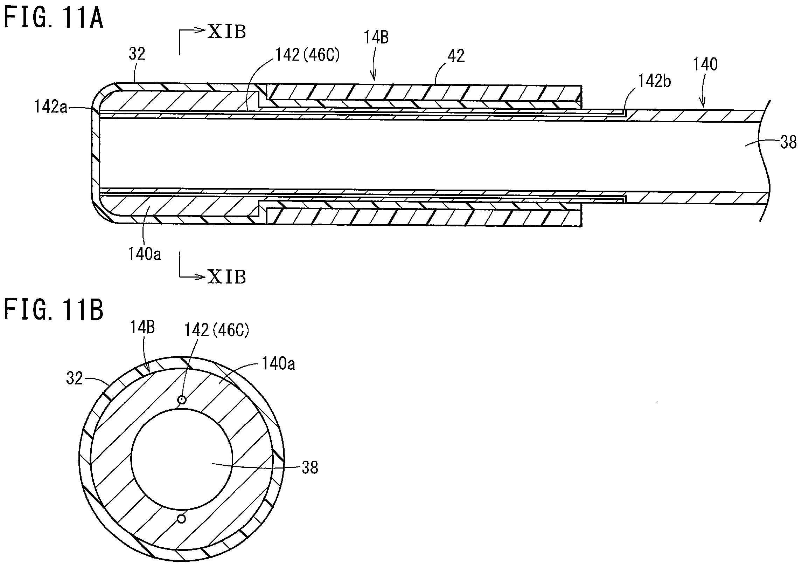

A gas discharge portion 46C of a placement device 14B according to a third modification example (not claimed) illustrated in Figs. 11A and 11B is configured to have an air discharge path 142 disposed in a shaft 140. The shaft 140 does not include the tip 40, and supports the filling balloon 32 by using a distal portion (enlarged diameter portion) of the shaft 140. The air discharge path 142 serves as a pair of vertical communication spaces formed in a cross-sectional circular shape inside a wall 140a of the shaft 140 which surrounds the flow path 38. Note that, the number, an arrangement location, or a shape of the air discharge paths 142 to be formed may be freely set.

One air discharge path 142 communicates with a distal opening portion 142a disposed on the distal surface of the shaft 140, extends in the direction toward the proximal end from the distal surface, is bent radially outward on the proximal side rather than the fixing tube 42, and communicates with a proximal opening portion 142b on the outer peripheral surface of the shaft 140. The distal opening portion 142a is closed by the deflated filling balloon 32. However, if the bone filling material C is supplied to the flow path 38, the air internally present in the flow path 38 pushes the filling balloon 32 and thus, the distal opening portion 142a is opened. Therefore, the air is guided to the proximal side of the filling balloon 32 via the air discharge path 142, and is discharged outward (for example, to the accommodation lumen 52 of the sheath 50) from the proximal opening portion 142b.

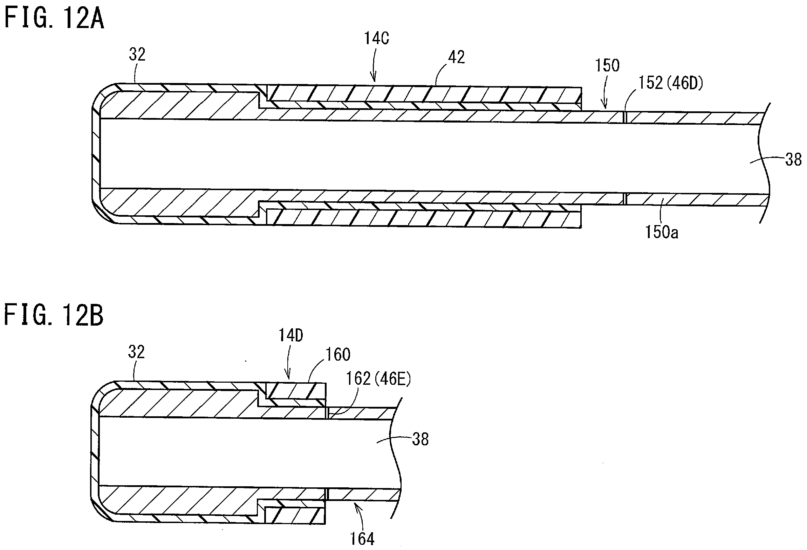

A gas discharge portion 46D of a placement device 14C according to a fourth modification example (not claimed) illustrated in Fig. 12A adopts a configuration in which a lateral hole 152 which serves as an air discharge path for the air is disposed in a shaft 150 (wall 150a) on the proximal side of the fixing tube 42. The lateral hole 152 can be formed to have a sufficiently small hole diameter to such an extent that the air is discharged and the bone filling material C does not leak outward. The lateral hole 152 is formed on the outer peripheral surface of the shaft 150. Therefore, it is possible to easily perform processing for the lateral hole 152. There is a possibility that the air remains inside the flow path 38 on the further distal side than the lateral hole 152. However, the remaining amount of the air is very small, and thus, rarely affects the cured bone filling material C.

A placement device 14D according to a fifth modification example (not claimed) illustrated in Fig. 12B is configured so that a fixing tube 160 is short and a lateral hole 162 (gas discharge portion 46E) is disposed in a position closer to a distal portion of a shaft 164. Therefore, the gas discharge portion 46E according to the fifth modification example can discharge the air present on the distal side of the flow path 38, more than the gas discharge portion 46D according to the fourth modification example.

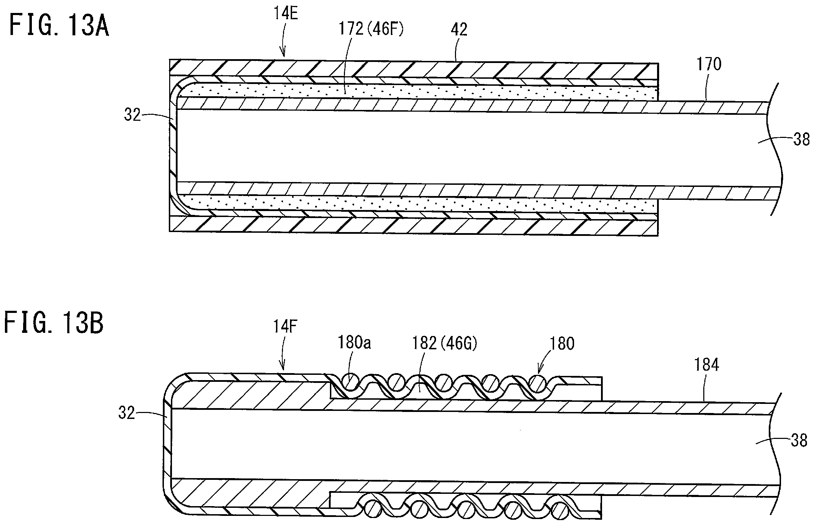

A placement device 14E according to a sixth modification example illustrated in Fig. 13A adopts a configuration in which a gas permeable membrane 172 (gas discharge portion 46F) is disposed in a distal portion (attachment portion) of a shaft 170 whose outer peripheral surface is formed smoothly. The gas permeable membrane 172 has a function as a gas permeable member for blocking circulation of the bone filling material C while permitting circulation of the air. For example, the gas permeable membrane 172 includes a porous material (PTFE, or the like), a mesh, a ceramic, and the like. The gas permeable membrane 172 is interposed between the filling balloon 32 and the shaft 170, and fixed by the fixing tube 42. If the bone filling material C is supplied to the flow path 38, the air internally present in the flow path 38 pushes the filling balloon 32, and is guided to the gas permeable membrane 172. The air is guided in the direction toward the distal end through the gas permeable membrane 172, and is discharged to the proximal side rather than the filling balloon 32.

A placement device 14F according to a seventh modification example illustrated in Fig. 13B adopts a configuration in which the filling balloon 32 is fixed by a coil member 180. Wiring rods 180a (linear portions) which configure the coil member 180 and are adjacent to each other compress the filling balloon 32 by a predetermined distance being apart from each other. The filling balloon 32 for fixing is fixed in such a manner that an attachment portion of the wiring rod 180a serves as a valley portion and a gap portion thereof serves as a ridge portion. Accordingly, an air discharge path 182 (gas discharge portion 46G) for the air is formed between an outer surface of a shaft 184 and an inner side of the ridge portion. Therefore, the air discharge path 182 has a spiral shape. The supply of the bone filling material C causes the air internally present in the flow path 38 to push the filling balloon 32. In this manner, the air is guided to the air discharge path 182, flows in a spiral shape through the air discharge path 182, and is discharged to the proximal side rather than the filling balloon 32.

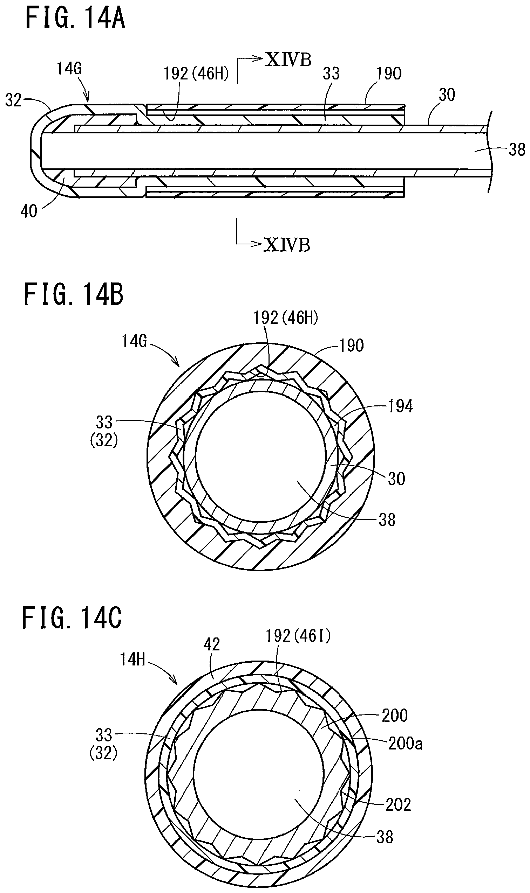

A gas discharge portion 46H of a placement device 14G according to an eighth modification example illustrated in Figs. 14A and 14B adopts a configuration in which a groove portion 192 is disposed on an inner peripheral surface of a fixing tube 190 which fixes the filling balloon 32 to the shaft 30. A fixing portion 33 of the filling balloon 32 left in an deflated state is configured so that the entire periphery thereof is in contact with the peripheral surface of the shaft 30. Then, if the bone filling material C is supplied and the air pushes the filling balloon 32, as illustrated in Fig. 14B, the groove portion 192 forms an air discharge path 194 by elastically deforming the fixing portion 33 radially outward. In this manner, the air is discharged to the proximal side rather than the fixing tube 190 via the air discharge path 194. The groove portion 192 is set so that the depth or the width is reasonably small. Accordingly, it is possible to prevent the bone filling material C from leaking out from the air discharge path 194.

In contrast to the groove portion 192 of the eighth modification example, a gas discharge portion 46I of a placement device 14H according to a ninth modification example illustrated in Fig. 14C adopts a configuration in which a groove portion 202 is disposed on an outer peripheral surface side of a shaft 200. In this case, the fixing portion 33 of the filling balloon 32 is attached to a ridge portion 200a of the shaft 200 which is formed between the groove portion 202 and the fixing portion 33 in a state where the filling balloon 32 is inflated. Therefore, it is possible to discharge the air inside the flow path 38 through the groove portion 202 between the filling balloon 32 and the shaft 200.

As another modification example of the bone treatment system 10 according to the present invention, the filling balloon 32 may be configured to have a bioabsorbable material, and the filling balloon 32 may also be placed together with the bone filling material C. The filling balloon 32 is absorbed with the lapse of time. Accordingly, it is possible to deploy the cured bone filling material C.

In the above-described embodiments, the filling balloon 32 is ruptured inside the bone after the bone filling material C is cured, that is, after the bone filling material C is in a state of being cured to such an extent so as not to be disintegrated by the body fluid. However, the filling balloon 32 may be ruptured inside the bone before the bone filling material C is in the state of being cured to such an extent so as not to be disintegrated by the body fluid. According to this method, a higher therapeutic effect can also be obtained as compared to a case where the bone filling material C is introduced into the bone without using the balloon.

Hitherto, the preferred embodiments of the present invention have been described as an example. However, the present invention is not limited to the above-described embodiments, and various modifications can be made within a scope without departing from the spirit of the present invention.

### Reference Signs List

10 bone treatment system
12 space forming device
14, 14A to 14H placement device
16, 16A deployment operation device
30, 120, 140, 150, 164, 170, 184, 200 shaft
32 filling balloon38 flow path
42, 160, 190 fixing tube
44, 122 air discharge tube
44a, 122a, 142, 182 air discharge path
44b, 122b, 130b, 142a distal opening portion
46, 46A to 461 gas discharge portion
70, 130 mandrel72 passage
80, 132 needle member
100 radius
108 space
130a penetrating path
152, 162 lateral hole
172 gas permeable membrane
180 coil member
192, 202 groove portion
C bone filling material

## Claims

1. A bone treatment system comprising a placement device (14) comprising: a bone filling material (c) a shaft (30) that has a lumen which enables the bone filling material (C) to be placed on a bone treatment site to flow therethrough;
a filling balloon (32) that is filled with the bone filling material (C) via the lumen and is disposed on a distal portion of the shaft (30);
a fixing member (42) that fixes the filling balloon (32) to the shaft (30); and
a gas discharge portion (46F to 46H) that causes gas inside the lumen to flow in a direction toward a proximal end rather than the balloon, when the lumen is filled with the bone filling material (C),
**characterized in that** the fixing member (42) surrounds the proximal portion of the filling balloon (32)and **in that** the gas discharge portion (46F to 46H) is configured to be formed between an outer surface of the shaft (30) and an inner side of the fixing member (42).

2. The bone treatment system according to Claim 1,
wherein the filling balloon (32) is broken in a state of maintaining attachment to the shaft (30), thereby exposing the bone filling material (C).

3. The bone treatment system according to Claim 1 or 2,
wherein the gas discharge portion (46F) is configured to have a gas permeable member which blocks a liquid or a solid, while allowing the gas to be permeable therethrough by being disposed between the shaft (30) and the filling balloon (32), in an attachment portion of the filling balloon (32) in the shaft (30).

4. The bone treatment system according to Claim 1 or 2, wherein the fixing member (42) is a tubular fixing member which covers the filling balloon (32), and
wherein the gas discharge portion (46H) is configured to have a groove portion (192) which is formed on an inner peripheral surface of the fixing member (190), and a fixing portion of the filling balloon (32) which is formed in a shape enabling the fixing portion to come into contact with the groove portion (192) along the groove portion (192).

5. The bone treatment system according to Claim 1 or 2,
wherein the fixing member (42) is a coil member (180) being wound up, and
wherein the gas discharge portion (46G) is configured to have a discharge path (182) which is formed in such a manner that an inner surface of the filling balloon (32) is separated from an outer surface of the shaft (30) in a gap of a linear portion configuring the coil member (180).

## Patentansprüche

1. Knochenbehandlungssystem, das eine Platzierungsvorrichtung (14) aufweist, umfassend:
ein Knochenfüllmaterial (C);
einen Schaft (30), der ein Lumen aufweist, der es ermöglicht, dass das Knochenfüllmaterial (C) an einer Knochenbehandlungsstelle angeordnet werden kann, um dort hindurchzufließen;
einen Füllballon (32), der über das Lumen mit dem Knochenfüllmaterial (C) gefüllt wird und an einem distalen Abschnitt des Schafts (30) angeordnet ist;
ein Befestigungselement (42), das den Füllballon (32) an dem Schaft (30) befestigt; und
einen Gasauslassabschnitt (46F bis 46H), der bewirkt, dass Gas im Inneren des Lumens eher in Richtung eines proximalen Endes als in Richtung des Ballons strömt, wenn das Lumen mit dem Knochenfüllmaterial (C) gefüllt ist,
**dadurch gekennzeichnet, dass** das Befestigungselement (42) den proximalen Abschnitt des Füllballons (32) umgibt und dadurch, dass der Gasauslassabschnitt (46F bis 46H) so konfiguriert ist, dass er zwischen einer Außenfläche des Schaftes (30) und einer Innenseite des Befestigungselements (42) ausgebildet ist.

2. Knochenbehandlungssystem nach Anspruch 1, wobei der Füllballon (32) in einem Zustand einer Aufrechterhaltung der Befestigung an dem Schaft (30) zerstört wird, wodurch das Knochenfüllmaterial (C) freigelegt wird.

3. Knochenbehandlungssystem nach Anspruch 1 oder 2, wobei der Gasauslassabschnitt (46F) so konfiguriert ist, dass er ein gasdurchlässiges Element, das eine Flüssigkeit oder einen Feststoff blockiert, während es dem Gas erlaubt dort hindurch permeabel zu sein, indem es zwischen dem Schaft (30) und dem Füllballon (32) angeordnet ist, in einem Befestigungsabschnitt des Füllballons (32) in dem Schaft (30) aufweist.

4. Knochenbehandlungssystem nach Anspruch 1 oder 2, wobei das Befestigungselement (42) ein rohrförmiges Befestigungselement ist, das den Füllballon (32) umschließt, und
wobei der Gasauslassabschnitt (46H) so konfiguriert ist, dass er einen Nutabschnitt (192), der an einer inneren Umfangsfläche des Befestigungselements (190) ausgebildet ist, und einen Befestigungsabschnitt des Füllballons (32) aufweist, der in einer Form ausgebildet ist, die es dem Befestigungsabschnitt ermöglicht, mit dem Nutabschnitt (192) entlang des Nutabschnitts (192) in Kontakt zu kommen.

5. Knochenbehandlungssystem nach Anspruch 1 oder 2, wobei das Befestigungselement (42) ein aufgewickeltes Spiralelement (180) ist, und
wobei der Gasauslassabschnitt (46G) so konfiguriert ist, dass er einen Auslasspfad (182) aufweist, der so ausgebildet ist, dass eine Innenfläche des Füllballons (32) von einer Außenfläche des Schafts (30) in einem Spalt eines linearen Abschnitts getrennt ist, der das Spiralelement (180) konfiguriert.

## Revendications

1. Système de traitement osseux comprenant un dispositif de placement (14) comprenant :
un matériau de remplissage osseux (C)
un arbre (30) qui a une lumière qui permet au matériau de remplissage osseux (C) d'être placé sur un site de traitement osseux pour s'écouler à travers celui-ci ;
un ballonnet de remplissage (32) qui est rempli du matériau de remplissage osseux (C) par l'intermédiaire de la lumière et est disposé sur une partie distale de l'arbre (30) ;
un élément de fixation (42) qui fixe le ballonnet de remplissage (32) à l'arbre (30) ; et
une partie d'évacuation de gaz (46F à 46H) qui amène un gaz à l'intérieur de la lumière à s'écouler dans une direction vers une extrémité proximale plutôt que le ballonnet, lorsque la lumière est remplie du matériau de remplissage osseux (C),
**caractérisé en ce que** l'élément de fixation (42) entoure la partie proximale du ballonnet de remplissage (32) et **en ce que** la partie d'évacuation de gaz (46F à 46H) est configurée pour être formée entre une surface externe de l'arbre (30) et un côté interne de l'élément de fixation (42).

2. Système de traitement osseux selon la revendication 1, dans lequel le ballonnet de remplissage (32) est rompu dans un état de maintien de la liaison à l'arbre (30), exposant ainsi le matériau de remplissage osseux (C).

3. Système de traitement osseux selon la revendication 1 ou 2,
dans lequel la partie d'évacuation de gaz (46F) est configurée pour avoir un élément perméable aux gaz qui bloque un liquide ou un solide, tout en permettant au gaz d'être perméable à travers celui-ci en étant disposé entre l'arbre (30) et le ballonnet de remplissage (32), dans une partie de liaison du ballonnet de remplissage (32) dans l'arbre (30).

4. Système de traitement osseux selon la revendication 1 ou 2, dans lequel l'élément de fixation (42) est un élément de fixation tubulaire qui recouvre le ballonnet de remplissage (32), et
dans lequel la partie d'évacuation de gaz (46H) est configurée pour avoir une partie de rainure (192) qui est formée sur une surface périphérique interne de l'élément de fixation (190), et une partie de fixation du ballonnet de remplissage (32) qui est formée dans une forme permettant à la partie de fixation de venir en contact avec la partie de rainure (192) le long de la partie de rainure (192).

5. Système de traitement osseux selon la revendication 1 ou 2,
dans lequel l'élément de fixation (42) est un élément de bobine (180) étant enroulé, et
dans lequel la partie d'évacuation de gaz (46G) est configurée pour avoir un trajet d'évacuation (182) qui est formé de telle manière qu'une surface interne du ballonnet de remplissage (32) est séparée d'une surface externe de l'arbre (30) dans un espace d'une partie linéaire configurant l'élément de bobine (180).
